# EUROPÄISCHE PATENTSCHRIFT

(11) **EP 0 402 908 B1**
(45) Veröffentlichungstag und Bekanntmachung des Hinweises auf die Patenterteilung: **18.05.1994**
(21) Anmeldenummer: 90111223.5
(22) Anmeldetag: 13.06.1990
(51) Int. Cl.: A61M 5/32

(54) **Injektionsspritze**
Injection syringe
Seringue à injections

(30) Priorität: 15.06.1989 KR 829089 U
(43) Veröffentlichungstag der Anmeldung: 19.12.1990
(73) Patentinhaber: Choul, Bang Young, Seoul (KR)
(72) Erfinder: Choul, Bang Young, Seoul (KR)
(74) Vertreter: Fincke, Karl Theodor, Dipl.-Phys. Dr.

(56) Entgegenhaltungen:
- WO-A-89/12475
- US-A- 4 692 156
- US-A- 4 747 830
- US-A- 4 861 338

## Beschreibung

Die Erfindung betrifft eine Injektionsspritze mit einem Zylinder, an dessen einem Ende ein eine Injektionsnadel halternder Nadelhalter anbringbar ist, mit einem in dem Zylinder verschiebbaren Kolben, an dem eine aus dem anderen Ende des Zylinders herausragende, an ihrem Ende mit einem Betätigungsgriff versehene Kolbenstange angebracht ist, und mit einer Vorrichtung zur selbsttätigen Verriegelung des über ein vorgegebenes Maß in Richtung zur Injektionsnadel vorgeschobenen Kolbens an dem Nadelhalter gegenüber Zurückziehen des Kolbens, bei der der Nadelhalter durch eine durch Hineindrehen und gegebenenfalls axiales Verschieben des Nadelhalters in den Zylinder zu lösende Drehverbindung mit dem Zylinder verbindbar ist und bei der sich an dem Nadelhalter und dem Kolben Drehsicherungsglieder befinden, die bei Vorschieben des Kolbens in Richtung zum Nadelhalter zur Verriegelung des Kolbens an dem Nadelhalter ineinandergreifen und durch Verdrehen des Betätigungsglieds eine Lösung der Drehverbindung und daraufhin ein Hineinziehen des Kolbens, des Nadelhalters und der Injektionsnadel über ihre ganze Länge in den Zylinder gestatten.

Eine Injektionsspritze dieser Art ist nach der US-PS 47 47 830 bekannt. Bei dieser Injektionsspritze sind die Drehsicherungsglieder durch zwei diametrale, in Längsrichtung des Kolbens verlaufende, radial vorstehende Flügel an einem pilzförmigen, zentralen, vorderen Ansatz des Kolbens gebildet und durch diesen Flügeln zugewandte, diametrale, innere Nuten im Nadelhalter. Der Benutzer muß daher den Kolben um bis zu 90° drehen, damit die Flügel in die Nuten eingreifen können.

Aufgabe der Erfindung ist es, ein besonders leichtes und sicheres Eingreifen der Drehsicherungsglieder des Kolbens in die des Nadelhalters zu ermöglichen, ohne den Kolben relativ zum Nadelhalter in eine bestimmte axiale Winkellage bringen zu müssen.

Zur Lösung dieser Aufgabe ist die Injektionsspritze eingangs genannter Art dadurch gekennzeichnet, daß die Drehsicherungsglieder Winkelabstände zueinander aufweisende, durch axiales Vorschieben des Kolbens in gegenseitigen, kämmenden Eingriff zu bringende Stege sind.

Nach einmaligem Gebrauch der Injektionsspritze wird deren Kolben in einer vorgeschobenen, einem entleerten Zustand entsprechenden Stellung gegen ein erneutes Zurückziehen zum Füllen und erneuten Gebrauch der Injektionsspritze verriegelt, so daß eine Wiederverwendung der Injektionsspritze nach einmaligem Gebrauch ausgeschlossen ist, und nach dem Gebrauch der Injektionsspritze wird ein Berühren der nunmehr insterilen und möglicherwiese mit Krankheitserregern behafteten Injektionsnadel über ihre ganze Länge einschließlich ihrer Spitze verhindert. Die Injektionsnadel an ihrem Nadelhalter kann also nach Gebrauch vollständig in den Zylinder hineingezogen werden, so daß die Injektionsnadel, insbesondere auch ihre Spitze, vom Zylinder schützend umgeben und praktisch unzugänglich ist. Der Kolben wird hierzu nach der Injektion mittels des mit ihmn verbundenen Betätigungsglieds verdreht, bis die Drehsicherungsglieder an ihm und an dem Nadelhalter ineinander eingreifen, woraufhin durch weiteres Verdrehen des Betätigungsglieds die Drehverbindung zwischen dem Nadelhalter und dem Zylinder gelöst wird. Die Injektionsnadel, der Nadelhalter und der Kolben sind nunmehr zu einer Einheit verbunden und können an dem Betätigungsglied so weit in den Zylinder hineingezogen werden, daß die Injektionsnadel einschließlich ihrer Spitze nicht mehr berührt werden kann und eine Infektion ausgeschlossen ist.

Um ein Herausziehen des Kolbens, des Nadelhalters und der Injektionsnadel aus dem hinteren, der Injektionsnadel abgewandten Ende des Zylinders, sowie ein erneutes Vorschieben nach vorne in die ursprüngliche Stellung, bei der die Injektionsnadel freiliegt, zu verhindern, ist die Injektionsspritze bevorzugt gekennzeichnet durch eine Vorrichtung zur selbsttätigen Verriegelung der über ein vorgegebenes Maß in den Zylinder gezogenen Kombination aus Kolben, Nadelhalter und Indjektionsnadel gegenüber Vorschieben dieser Kombination.

Eine besonders einfache und ohne zusätzliche Bauteile besonders funktionssichere Ausführung der Verriegelungseinrichtung zwischen dem Kolben und dem Nadelhalter bzw. zwischen dem Kolben und dem Zylinder ergibt sich, wenn wenigstens eine der Vorrichtungen zur selbsttätigen Verriegelung durch wenigstens einen nasenförmigen Vorsprung und eine zu diesem komplementäre, nasenförmige Vertiefung gebildet ist, die in bezug zueinander nachgiebig sind.

Eine schnelle Montage und sichere Befestigung der Injektionsnadel an dem Nadelhalter erlaubt und ein versehentliches Lösen der Drehverbindung zwischen dem Nadelhalter und dem Zylinder verhindert eine Ausführung, bei der die Injektionsnadel mit dem Nadelhalter durch eine durch gegenüber der erstgenannten Drehverbindung gegensinniges Herausdrehen und gegebenenfalls axiales Verschieben der Injektionsnadel aus dem Nadelhalter lösbare Drehverbindung verbindbar ist.

Eine lösbare und gleichzeitig gegen den Flüssigkeitsdruck im Inneren der Injektionsspritze während der Injektion sicher abdichtende Drehverbindung besteht, wenn wenigstens eine der Drehverbindungen eine Schraubverbindung ist.

Im folgenden wird die Erfindung an zwei Ausführungsbeispielen unter Hinweis auf die beigefügten Zeichnungen beschrieben. Es zeigen:
Fig. 1 einen Längsschnitt durch das Vorderteil einer Injektionsspritze gemäß einem ersten Ausführungsbeispiel;
Fig. 2 einen Längsschnitt durch die Injektionsspritze nach Fig. 1 mit weit herausgezogenem Kolben;
Fig. 3 einen Längsschnitt durch die Injektionsspritze nach Fig. 1 nach der Injektion;
Fig. 4 einen Längsschnitt durch das Vorderteil der Injektionsspritze nach Fig. 1 nach der Injektion mit in dem Nadelhalter verrastetem Kolben;
Fig. 5 einen Längsschnitt durch das Vorderteil der Injektionsspritze nach Fig. 1 nach dem Lösen des Nadelhalters von dem Zylinder;
Fig. 6 einen Längsschnitt durch die Injektionsspritze nach Fig. 1 mit zusammen mit dem Nadelhalter und der Injektionsnadel in den Zylinder zurückgezogenem, in dem Zylinder verrastetem Kolben;
Fig. 7 einen Längsschnitt durch das Vorderteil einer Injektionsspritze gemäß einem zweiten Ausführungsbeispiel mit in dem Nadelhalter verrastetem Kolben.

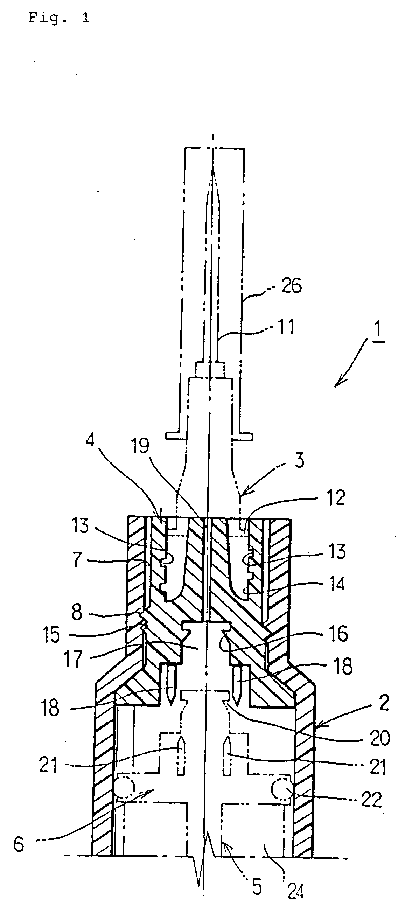

Die Injektionsspritze 1 nach Fig. 1 bis 6 weist einen Zylinder 2 konstanten Durchmessers auf, in dem eine Kolbenstange 5 verschiebbar geführt ist, eine Nadelhaltehülse 3 für eine Injektionsnadel 11, einen Nadelhalter 4 für die Nadelhaltehülse 3 und einen Kolben 6, der mit der Kolbenstange 5 einstückig ist. Der Zylinder 2 ist in einem verengten vorderen Bereich mit einem in seine Innenwand 7 geformten Innengewindeabschnitt 8 versehen, in den der Nadelhalter 4 eingeschraubt ist. In das hintere Teil des Zylinders 2 ist ein nasenförmiger Verriegelungsvorsprung 9 eingeformt, in dem nasenförmige Verriegelungsvertiefungen 25 in im Querschnitt kreuzförmig angeordneten Führungsrippen 24 an der Kolbenstange 5 einrastbar sind. Am hinteren Ende des Zylinders 2 befinden sich außen Fingerwiderlager 10 für einen runden Betätigungsgriff 23 am freien Ende der Kolbenstange 5, mittels dem die Kolbenstange 5 verdreht und verschoben werden kann.

In dem vorderen Ende der Nadelhaltehülse 3 ist die Injektionsnadel 11 unverrückbar befestigt. An das hintere Ende der Nadelhaltehülse 3 ist außen ein Flansch 12 angeformt. In eine Innenwandfläche 14 des Nadelhalters 4 ist ein Linksgewindeabschnitt 13 eingeformt, in den der Flansch 12 der Nadelhaltehülse 3 zu schrauben ist. Ein in den Innengewindeabschnitt 8 des Zylinders 2 von Hand oder maschinell geschraubter Rechtsgewindeabschnitt 15 ist in den Außenumfang des hinteren Mittelteils des Nadelhalters 4 eingeformt.

Der Nadelhalter 4 ist hinten mit einer Höhlung 17 versehen. Ein an den Nadelhalter 4 angeformter nasenförmiger Verriegelungsvorsprung 16 steht in die Höhlung 17 hervor. Von der die Höhlung 17 hinten umgebenden Innenwand des Nadelhalters 4 stehen radial angeformte stegförmige Drehsicherungsglieder 18 hervor. Das vordere Mittelteil des Nadelhalters 4 ist von einem Kanal 19 durchsetzt, durch den zu injizierende Flüssigkeit zur Injektionsnadel 11 gelangt.

In eine nasenförmige Verriegelungsvertiefung 20 an einem vorderen Ansatz des Kolbens 6 ist der nasenförmige Verriegelungsvorsprung 16 in der Höhlung 17 des Nadelhalters 4 nachgiebig einrastbar. Die Verriegelungsvertiefung 20, die mit dem Verriegelungsvorsprung 16 des Nadelhalters 4 verriegelbar ist, und stegförmige Drehsicherungsglieder 21 zum kämmenden Eingriff mit den Drehsicherungsgliedern 18 des Nadelhalters 4 stehen radial von dem Außenumfang des Kolbens 6 hervor. An dem Umfang des Kolbens 6 ist ein dicht an der Innenwand 7 des Zylinders 2 anliegender O-Ring 22 aus Gummi befestigt.

Die kreuzförmig angeordneten Führungsrippen 24 und die Kolbenstange 5 sind an dem runden Betätigungsgriff 23 festgeklebt. Die nasenförmigen Verriegelungsvertiefungen 25 im Kolben 6 verrasten nachgiebig mit dem nasenförmigen Verriegelungsvorsprung 9 im Zylinder 2, wenn der Kolben 6 nach hinten gezogen wird, und verhindern ein vollständiges Herausziehen der Kolbenstange 5 aus dem Zylinder 2.

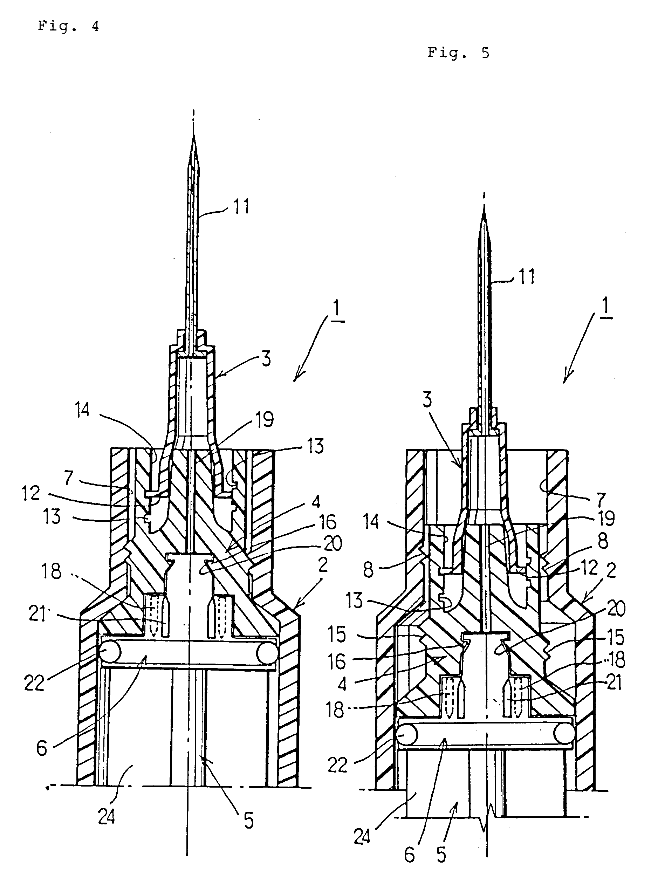

Vor einem Gebrauch wird der Nadelhalter 4 mit der Nadelhaltehülse 3 gegensinnig zur Schraubverbindung zwischen dem Nadelhalter 4 und dem Zylinder 2 verschraubt, wonach eine in Fig. 1 strichpunktiert angedeutete Nadelkappe 26 abgenommen wird. Wenn eine Injektionsflüssigkeit durch Vorbewegen des Kolbens 6 in einen Körper injiziert worden ist, befindet sich der Kolben 6 etwa in der in Fig. 3 dargestellten Position. In dieser Position wird die Injektionsnadel 11 aus dem Körper herausgezogen und dann der Kolben 6 weiter nach vorne in die Position gemäß Fig. 4 geschoben, wodurch die Verriegelungsvertiefung 20 des Kolbens 6 in den Verriegelungsvorsprung 16 in der Höhlung 17 des Nadelhalters 4 eingreift, so daß der Kolben 6 mit dem Nadelhalter 4 innerhalb des Zylinders 2 verbunden wird.

Fig. 7 zeigt ein zweites Ausführungsbeispiel, bei dem gegenüber dem ersten Ausführungsbeispiel der Rechtsgewindeabschnitt 15 im äußeren Umfang des Nadelhalters 4, der in den Innengewindeabschnitt 8 des Zylinders 2 eingeschraubt wird, vereinfacht ausgebildet ist. Im Unterschied zum ersten Ausführungsbeispiel ist kein Innengewindeabschnitt 13 in einer Innenwandfläche 14 des Nadelhalters 4 vorgesehen. Vielmehr ist ein Ansatz 27 an den Nadelhalter 4 angeformt, auf den die Nadelhaltehülse 3 dicht und unverrückbar aufzustecken ist.

Im folgenden wird die Handhabung und Funktionsweise der Injektionsspritze 1 nach dem ersten Ausführungsbeispiel beschrieben.

Zur Schraubverbindung des Nadelhalters 4 mit dem Zylinder 2 steckt man den Nadelhalter 4 in das hintere Ende des Zylinders 2, drückt ihn dann nach vorne und dreht ihn dann etwa eine viertel bis eine volle Umdrehung im Uhrzeigersinn nach rechts, entweder manuell oder maschinell, bis der Innengewindeabschnitt 8 an der Innenwand 7 des Zylinders 2 den Rechtsgewindeabschnitt 15 am Außenumfang des Nadelhalters 4 fest umgriffen hat. Dann schraubt man den Flansch 12 an der Nadelhaltehülse 3 in den Linksgewindeabschnitt 13 des Nadelhalters 4. Die Schraubverbindungsrichtung zwischen der Injektionsnadel 11 und dem Nadelhalter 4 ist dabei also entgegengesetzt zur Schraubverbindungsrichtung zwischen dem Zylinder 2 und dem Nadelhalter 4.

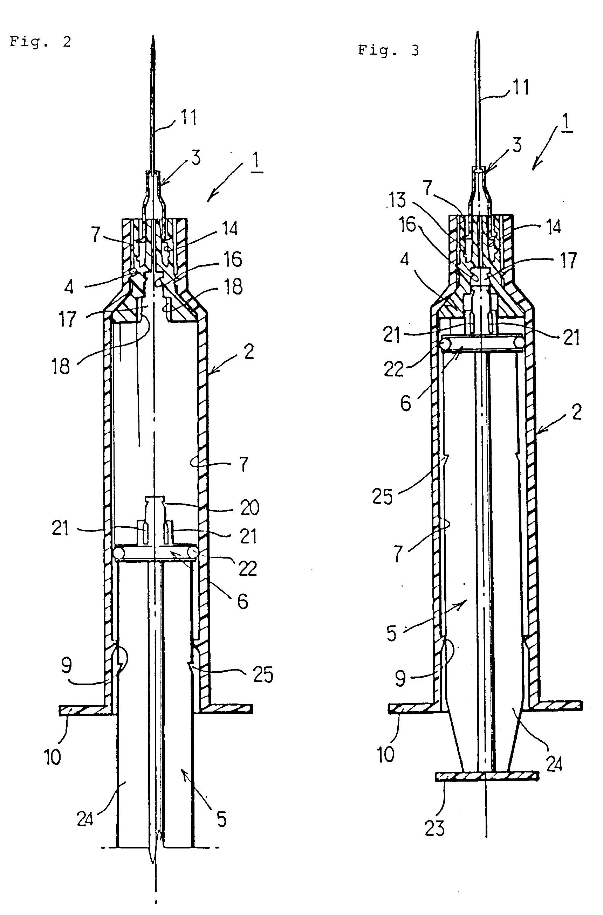

Nachdem die Nadelhaltehülse 3 eingesetzt ist und nachdem die Nadelkappe 26, die die Injektionsnadel 11 entsprechend der strichpunktierten Linie in Fig. 1 bedeckt, entfernt ist, wird die Injektionsnadel 11 in einen Körper eingestochen, und dann wird der Kolben 6 mittels der Kolbenstange 5 nach vorne aus der Position gemäß Fig. 2 in die Position gemäß Fig. 3 geschoben.

Nachdem die Injektion durchgeführt worden ist, wird die Injektionsnadel 11 aus dem Körper herausgezogen und mittels des Betätigungsgriffs 23 die Kolbenstange 5 in die Position gemäß Fig. 4 nach vorne geschoben, bis also die stegförmigen Drehsicherungsglieder 21 an dem vorderen Ansatz des Kolbens 6 kämmend zwischen die stegförmigen Drehsicherungsglieder 18 am Nadelhalter 4 greifen und der vordere Ansatz des Kolbens 6 in die Höhlung 17 des Nadelhalters 4 eingedrückt ist. Der nasenförmige Verriegelungsvorsprung 16 am Nadelhalter 4 und die nasenförmige Verriegelungsvertiefung 20 im vorderen Ansatz des Kolbens 6 greifen dann ineinander. Diese Art der Verriegelung zwischen dem Verriegelungsvorsprung 16 und der Verriegelungsvertiefung 20 verbindet den Kolben 6 fest mit dem Nadelhalter 4 und verhindert eine Wiederverwendung der Injektionsspritze 1.

Wenn der Kolben 6 gegen den Uhrzeigersinn gedreht wird, gelangen die stegförmigen Drehsicherungsglieder 18 in der Höhlung 17 des Nadelhalters 4 in Kontakt mit den stegförmigen Drehsicherungsgliedern 21 am Ansatz des Kolbens 6. Durch weiteres Drehen des Kolbens 6 um etwa eine viertel bis eine volle Umdrehung wird die Schraubverbindung zwischen dem Innengewindeabschnitt 8 im Zylinder 2 und dem Rechtsgewindeabschnitt 15 am Nadelhalter 4 gemäß Fig. 5 gelöst.

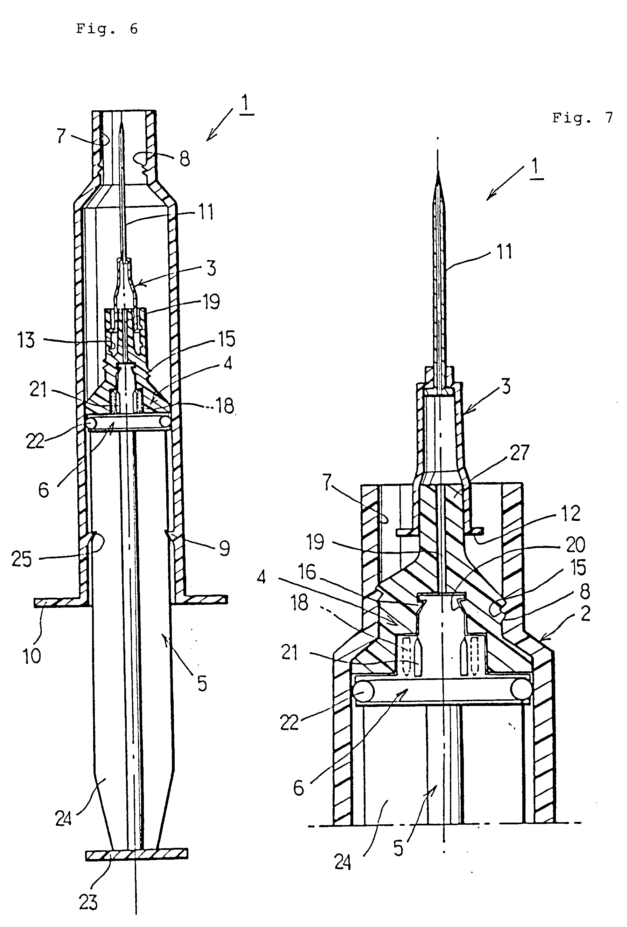

Dann wird der Kolben 6 nach hinten gezogen, bis der nasenförmige Verriegelungsvorsprung 9 an der Innenwand 7 des Zylinders 2 in die nasenförmigen Verriegelungsvertiefungen 25 in den kreuzförmig angeordneten Führungsrippen 24 einrastet. Ein Verschieben der Nadelhaltehülse 3, des Nadelhalters 4 und des Kolbens 6 aus der Position gemäß Fig. 6 wird dadurch verhindert.

Die Injektionsnadel 11 liegt dabei vollständig innerhalb des Zylinders 2, so daß eine Infektion durch sie ausgeschlossen wird.

Handhabung und Funktionsweise der Injektionsnadel 1 nach dem zweiten Ausführungsbeispiel sind bis auf die Steckverbindung zwischen der Nadelhaltehülse 3 und dem vorspringenden Ansatz 27 am Nadelhalter 4 analog.

## Patentansprüche

1. Injektionsspritze (1) mit einem Zylinder (2), an dessen einem Ende ein eine Injektionsnadel (11) halternder Nadelhalter (4) anbringbar ist, mit einem in dem Zylinder (2) verschiebbaren Kolben (6), an dem eine aus dem anderen Ende des Zylinders (2) herausragende, an ihrem Ende mit einem Betätigungsgriff (23) versehene Kolbenstange (5) angebracht ist, und mit einer Vorrichtung zur selbsttätigen Verriegelung des über ein vorgegebenes Maß in Richtung zur Injektionsnadel (11) vorgeschobenen Kolbens (6) an dem Nadelhalter (4) gegenüber Zurückziehen des Kolbens (6), bei der der Nadelhalter (4) durch eine durch Hineindrehen und gegebenenfalls axiales Verschieben des Nadelhalters (4) in den Zylinder (2) zu lösende Drehverbindung (8, 15) mit dem Zylinder (2) verbindbar ist und bei der sich an dem Nadelhalter (4) und dem Kolben (6) Drehsicherungsglieder (18, 21) befinden, die bei Vorschieben des Kolbens (6) in Richtung zum Nadelhalter (4) zur Verriegelung des Kolbens (6) an dem Nadelhalter (4) ineinandergreifen und durch Verdrehen des Betätigungsglieds (23) eine Lösung der Drehverbindung (8, 15) und daraufhin ein Hineinziehen des Kolbens (6), des Nadelhalters (4) und der Injektionsnadel (11) über ihre ganze Länge in den Zylinder (2) gestatten, **dadurch gekennzeichnet,** daß die Drehsicherungsglieder (18, 21) Winkelabstände zueinander aufweisende, durch axiales Vorschieben des Kolbens (6) in gegenseitigen, kämmenden Eingriff zu bringende Stege (18, 21) sind.

2. Injektionsspritze nach Anspruch 1, gekennzeichnet durch eine Vorrichtung (9, 25) zur selbsttätigen Verriegelung der über ein vorgegebenes Maß in den Zylinder (2) gezogenen Kombination (6, 4, 11) aus Kolben (6), Nadelhalter (4) und Injektionsnadel (11) gegenüber Vorschieben dieser Kombination (6, 4, 11).

3. Injektionsspritze nach einem der vorstehenden Ansprüche, dadurch gekennzeichnet, daß wenigstens eine der Vorrichtungen (16, 20; 9, 25) zur selbsttätigen Verriegelung durch wenigstens einen nasenförmigen Vorsprung (16; 9) und eine zu diesem komplementäre, nasenförmige Vertiefung (20; 25) gebildet ist, die in bezug zueinander nachgiebig sind.

4. Injektionsspritze nach einem der Ansprüche 1 bis 3, dadurch gekennzeichnet, daß die Injektionsnadel (11) mit dem Nadelhalter (4) durch eine durch gegenüber der erstgenannten Drehverbindung (8, 15) gegensinniges Herausdrehen und gegebenenfalls axiales Verschieben der Injektionsnadel (11) aus dem Nadelhalter (4) lösbare Drehverbindung (12, 13) verbindbar ist.

5. Injektionsspritze nach einem der Ansprüche 1 bis 4, daduch gekennzeichnet, daß wenigstens eine der Drehverbindungen (8, 15; 12, 13) eine Schraubverbindung ist.

## Claims

1. An injection syringe (1) comprising a cylinder (2) to one end of which can be attached a needle holder (4) for supporting an injection needle (11),
a piston (6) movable within said cylinder (2) and to which is attached a piston rod (5) projecting from the other end of said cylinder (2) and being provided with a manipulating handle (23) at the end thereof, and
means for automatically securing said piston (6) to said needle holder (4) so as to prevent retraction thereof when said piston is advanced a predetermined amount in the direction of said injection needle (11),
wherein said needle holder (4) is connectable to said cylinder (2) by a rotary connection (8, 15) which can be released by rotation and, if required, by axial displacement of said needle holder (4) in said cylinder (2), and wherein rotation preventing members (18, 21) are provided on said needle holder (4) and on said piston (6) which members engage with one another upon advance of said piston (6) in the direction of said needle holder (4) for securing said piston (6) to said needle holder (4), whereby turning said manipulating handle (23) permits the release of the rotary connection (8, 15) followed by the withdrawal of said piston (6), said needle holder (4), and said injection needle (11) over their length into said cylinder (2),
characterised in that said rotation preventing members (18, 21) are angularly spaced apart spines (18, 21) arranged to be brought into mutual meshing engagement by axially advancing said piston (6).

2. An injection syringe according to claim 1 characterised by means (9, 25) for automatically locking the combination (6, 4, 11) of piston (6), needle holder (4), and injection needle (11), when drawn into said cylinder (2) by a predetermined amount, against advance of said combination (6, 4, 11).

3. An injection syringe according to any of the preceding claims, characterised in that at least one of said means (16, 20; 9, 25) for automatically securing is formed by at least one lug-shaped protrusion (16; 9) and a complementary lug-shaped groove (20; 25) which are resilient relative to one another.

4. An injection syringe according to any of claims 1 to 3, characterised in that said injection needle (11) is engageable with said needle holder (4) by a rotary connection (12, 13) which is releasable from said needle holder by a rotation in the opposite direction from the first-mentioned rotary connection (8, 15) and, if required, axial displacement of the injection needle (11).

5. An injection syringe according to any of the claims 1 to 4, characterised in that at least one of said rotary connections (8, 15; 12, 13) is a screw connection.

## Revendications

1. Seringue à injections (1) comprenant un cylindre (2) à une extrémité duquel peut être monté un porte-aiguille (4) supportant une aiguille d'injection (11), un piston (6) pouvant être déplacé dans le cylindre (2), sur lequel est montée une tige de piston (5) faisant saillie à l'extérieur de l'autre extrémité du cylindre (2) et munie à son extrémité d'une poignée d'actionnement (23), et un dispositif de verrouillage automatique du piston (6) sur le porte-aiguille (4) quand il est avancé sur une distance prédéterminée en direction de l'aiguille d'injection (11) et qui empêche le piston (6) de revenir en arrière, seringue dans laquelle le porte-aiguille (4) peut être relié au cylindre (2) par vissage et éventuellement déplacement axial du porte-aiguille (4) dans le cylindre (2) pour constituer une liaison par rotation (8, 15) dégageable, et dans laquelle sont prévus des organes de blocage de rotation (18, 21) sur le porte-aiguille (4) et sur le piston (6), qui viennent s'engager les uns dans les autres lors du déplacement vers l'avant du piston (6) en direction du porte-aiguille (4) pour verrouiller le piston (6) sur le porte-aiguille (4) et qui permettent par rotation de l'organe d'actionnement (23) un dégagement de la liaison par rotation (8, 15) et ensuite la rentrée du piston (6), du porte-aiguille (4) et de l'aiguille d'injection (11) sur la totalité de leur longueur dans le cylindre (2), caractérisée en ce que les organes de blocage de rotation (18, 21) sont constitués par des nervures (18, 21) disposées selon un certain écart angulaire les unes par rapport aux autres et amenées en prise mutuelle par emboîtement par un déplacement axial vers l'avant du piston (6).

2. Seringue à injections selon la revendication 1, caractérisée par un dispositif (9, 25) de verrouillage automatique de la combinaison (6, 4, 11) constituée par le piston (6), le porte-aiguille (4) et l'aiguille d'injection (11) quand elle est tirée sur une distance prédéterminée dans le cylindre (2), qui s'oppose à un déplacement vers l'avant de cette combinaison (6, 4, 11).

3. Seringue à injections selon l'une quelconque des revendications précédentes, caractérisée en ce qu'au moins l'un des dispositifs (16, 20; 9, 25) de verrouillage automatique est formé par au moins une saillie en forme de nez (16; 9) et un évidement en forme de nez (20; 25) complémentaire à la saillie, qui sont flexibles l'un par rapport à l'autre.

4. Seringue à injections selon l'une quelconque des revendications 1 à 3, caractérisée en ce que l'aiguille d'injection (11) peut être reliée au porte-aiguille (4) au moyen d'une liaison à rotation dégageable (12, 13) par une rotation en sens contraire à la liaison par rotation (8, 15) premièrement mentionnée et éventuellement par un déplacement axial de l'aiguille d'injection (11) hors du porte-aiguille (4).

5. Seringue à injections selon l'une quelconque des revendications 1 à 4, caractérisée en ce qu'au moins l'une des liaisons par rotation (8, 15; 12, 13) est constituée par une liaison par vissage.
